# EUROPEAN PATENT APPLICATION

(11) **EP 4 327 848 A1**
(43) Date of publication of application: **28.02.2024**
(21) Application number: 22191501.0
(22) Date of filing: 22.08.2022
(51) Int. Cl.: A61M 5/30, A61M 5/44

(54) **METHOD FOR INJECTING A LIQUID SUBSTANCE AND APPARATUS**

(71) Applicant: Otto-von-Guericke-Universität Magdeburg, 39106 Magdeburg (DE)
(72) Inventor: Rosselló, Dr. Juan Manuel, 39106 Magdeburg (DE); Ohl, Prof. Dr. Claus-Dieter, 39128 Magdeburg (DE)

(57) **Abstract**

The invention relates to a method for injecting a liquid substance into a material, wherein a frozen projectile is formed and creates a channel in the material, and the liquid substance is released into the channel. The invention further relates to a corresponding apparatus.

## Description

The invention relates to a method for injecting a liquid substance into a material and to a corresponding apparatus.

Liquid substances have to be inserted into materials for a variety of purposes. For example, liquid drugs like vaccines or drugs for treatment of a specific disease have to be inserted into biological tissue like human tissue or animal tissue. It is common practice to use needle injectors for this purpose, wherein such needle injectors are typically disposed immediately after one single use. This generates an enormous amount of waste and of need for raw materials and energy for producing such needle injectors. In addition, many people are afraid of such needle injectors and do not like the feeling of having such injectors being inserted into their bodies.

It is thus an object of the present invention to provide for a method for injecting a liquid substance into a material that is alternative or improved with respect to the prior art. It is a further object of the invention to provide for a corresponding apparatus.

This is solved by a method and an apparatus according to the respective main claims. Preferred embodiments can, for example, be derived from the respective dependent claims. The content of the claims is made a content of the application by explicit reference.

The invention relates to a method for injecting a liquid substance into a material. The method comprises the following steps:
- Providing the liquid substance in a pressure chamber, the pressure chamber having an opening,
- Freezing a part of the liquid substance, or another liquid, in the pressure chamber adjacent to the opening, thereby forming a frozen projectile closing the opening,
- Applying a pressure to the liquid substance in the pressure chamber,
- Positioning the opening besides the material,
- Releasing the projectile, so that the pressure in the pressure chamber drives the projectile out of the opening, the projectile forming a channel in the material,
- Releasing liquid substance from the pressure chamber out of the opening into the channel.

With the inventive method, a liquid substance can be injected into a material, for example a liquid drug can be injected into biological tissue, without the need to insert a needle into the material and without any component that has to be disposed immediately after use. The method can be performed by an apparatus, for example an apparatus as described further below, and this apparatus can be used indefinitely without the risk of transferring diseases between people. It does not have to contact the material. For example, it does not have to contact a human skin, in order to inject a liquid substance into the skin. Instead, a channel is formed by a projectile and the channel is used in order to inject the liquid substance in a specific depth of the material. The projectile can be formed instantaneously at the point of use and it can remain in the material, as it is typically made of the same liquid substance that has to be injected, or it can be made of a non-critical substance like water. Patients being afraid of needle injectors may better accept injections with the inventive method.

The formation of the projectile ensures that a channel is formed in the tissue. This channel allows the liquid substance to freely reach a certain depth. Compared with solutions that do not use a projectile, using a projectile significantly increases reliability and prevents uncontrolled spilling of the liquid substance, which can especially be produced by back-splashing and liquid recirculation outside the targeted area.

For example, a liquid substance may be a liquid drug. This liquid drug may especially be a mixture of water, alcohol and/or another solvent, and a biologically active component like a vaccine or a curing drug. However, also other liquid substances can be injected using the inventive method. The material can, for example, be a biological tissue, for example, a human skin. However, the method can equally be applied for animals or for injecting a liquid substance into some technical material.

The pressure chamber is typically adapted to increase the pressure of the liquid substance contained in the pressure chamber, in order to drive a projectile formed as described herein. The opening is typically an opening out of which the projectile will leave the pressure chamber. Especially, an overpressure can be generated in the pressure chamber. This overpressure may be generated with respect to the surrounding air pressure. Suitable means for increasing a pressure are described further below.

The opening may be formed integral with the rest of the pressure chamber. However, the opening may also be separate or may be formed at a separate element that is exchangeable with respect to the rest of the pressure chamber. For example, a needle portion may be formed in such an element. This allows adapting the opening to specific needs.

The liquid substance may be frozen itself in order to form the projectile. However, also another liquid can be used in order to form the projectile. The liquid substance itself can especially be used if the liquid substance can be frozen without loss of quality, which is the case, for example, for most liquid drugs. For example, most vaccines have to be kept under low temperatures for storage anyway and will not degrade when being frozen. If the liquid substance cannot be frozen without deterioration, another liquid can be used, for example water or a sterile solution. Such liquids typically do not generate any unwanted biological or technical effect when being injected into the material. The freezing may typically be performed on only a part of the liquid substance, especially only a small part like up to one percent, up to two percent, up to five percent or up to ten percent of the liquid substance contained in the pressure chamber. The freezing may especially be performed by providing a cold temperature locally at a zone in which the freezing should be performed. For example, means described below can be used in order to generate a cold temperature.

If another liquid is used, it may be sucked through the opening into the pressure chamber for freezing, or it may be provided from the side of the main part of the pressure chamber. Sucking may be realized by providing an underpressure in the pressure chamber. It can be frozen separately before introducing the liquid to be injected into the material. This prevents mixing of the different liquids. However, it may also be frozen after the pressure chamber was filled with the liquid to be injected.

The step of applying pressure to the liquid substance in the pressure chamber may especially comprise increasing the pressure in the pressure chamber, especially generating an overpressure. The pressure generated before releasing the projectile can especially be used in order to adapt the intended depth of the channel. This will be described further below.

For applying or increasing pressure, means as described further below can be used.

The opening can especially be positioned besides the material such that the projectile, when being released from the pressure chamber, generates the channel at a specific position and with a specific direction. The direction can, for example, be adapted by suitable orienting the pressure chamber and/or the opening.

The releasing of the projectile especially means that the pressure in the pressure chamber exerts a force on the projectile which is greater than holding forces holding the projectile in place, especially so that the projectile accelerates and is driven out of the opening. Then, it has a certain kinetic energy in order to generate the channel in the material. After having formed this channel, the projectile typically melts and the liquid, out of which the projectile was formed, remains in the material. This is typically no problem, as the projectile is to be formed of the liquid substance which should deliberately be injected anyway, or is at least formed of a non-critical liquid.

After the channel was formed, the liquid substance can use this channel in order to flow to a certain depth, which is given by the depth of the channel. No needle is needed in order to bring the liquid substance in the material. After the injection, the channel typically closes in a short amount of time, as typical diameters of such channels are quite small. For example, biological tissue like a human skin is typically able to deform for a moment and recover after a short period due to its elastic properties and close such channels almost instantaneously.

Especially, the freezing of the part of the liquid substance may be performed by temporarily placing the opening on a cooling device, or by using a cooling device being fixed to the pressure chamber. The cooling device may especially be, or comprise, a thermo-electric element. Such a thermo-electric element may especially use the Peltier effect. This can be used in order to efficiently generate cold temperatures. The cooling device may present a surface, and the opening can be placed on this surface temporarily in order to freeze a part of the liquid substance being contained in the pressure chamber or in order to freeze another liquid. However, the cooling device can also be integrated into an apparatus containing the pressure chamber. For example, the cooling device can surround the opening to locally generate cold temperatures in order to freeze a liquid. As an alternative to using a thermo-electric element for the cooling device, a Carnot cycle apparatus can be used, for example.

Forming the projectile adjacent to the opening may especially mean that the projectile is formed with no distance or in a distance to the opening so that the projectile can be ejected from the opening without an obstruction that could harm the ejection of the projectile. Especially, a cross section of the pressure chamber may be constant from the position at which the projectile is formed to the opening.

According to an implementation, releasing the projectile may comprise at least partially melting the projectile at its contact portion to the pressure chamber or to the opening. This can reduce a holding force holding the projectile in place, so that an overpressure in the pressure chamber is able to drive the projectile out of the opening when the holding force becomes lower than the force exerted on the projectile by the overpressure. In an alternative implementation, the overpressure can be increased until the force exerted on the projectile by the overpressure is larger than the holding force. It is also possible to combine both implementations.

Releasing the projectile may especially comprise locally heating a region at which the projectile is positioned, so that the projectile is at least partially melted at its contact portion to the pressure chamber or to the opening. This can reduce the holding force as described above.

For example, the partially melting and/or the locally heating may be performed by a heating device, an electric heater and/or by a solenoid. Especially, a solenoid can be used as an electric heater. Such an electric heater is a simple and reliable means in order to locally increase a temperature so that the projectile partially melts.

Releasing the projectile may especially comprise pressing the projectile out by overpressure in the pressure chamber. This overpressure can especially be generated by means as described further below. The overpressure typically presses the projectile out of the opening when a force exerted on the projectile generated by the overpressure exceeds a holding force, wherein such a holding force is typically generated by a friction and/or by a pressure exerted by the projectile in a radially outwards direction. Especially, such a holding force may be generated already due to the fact that typical liquids like water increase in seize when being frozen, so that the projectile has a larger volume compared with the liquid being used in order to generate the projectile. Such an increased volume automatically generates a frictional force between the projectile and a surrounding portion of the pressure chamber.

Especially, the opening may be kept out of contact with the material at least during performing the method. Furthermore, the opening may be kept out of contact with the material generally during usage of the pressure chamber or a corresponding apparatus for injecting a liquid substance. This can avoid contamination of the opening or other parts of an apparatus used for injecting the liquid, and can avoid that diseases are spread between patients, even if the same apparatus is used for injecting liquids into different patients. Especially, the opening can be placed in a distance to the surface of the material that allows the liquid substance to substantially enter the channel.

The material may especially be biological tissue. For example, it may be a human or animal skin. The liquid substance may especially be a liquid drug. Especially for the specific case of injecting a liquid drug, for example a vaccine, into human tissue, the method has specific advantages of significantly reducing consumption of raw materials and energy for producing disposable needle injectors, and it also reduces significantly the amount of waste generated for drug injection. In principle, an apparatus used for performing the method can be used indefinitely and should only be disposed when showing a substantial malfunction that cannot be repaired.

According to an implementation, the pressure may be applied to the liquid substance in the pressure chamber by introducing more liquid substance. This additional liquid substance may increase the pressure because the volume inside the pressure chamber is limited, so that additional liquid substance leads to a pressure increase.

According to an implementation, the pressure may be applied to the liquid substance by introducing compressed gas. This compressed gas may especially exert a force on the liquid. As a consequence, the pressure on the liquid will match the higher pressure of the gas.

According to an implementation, the pressure may be applied to the liquid substance by decreasing a volume of the pressure chamber. This can increase the pressure even if the amount of liquid substance and/or gas inside the pressure chamber is kept constant.

For example, the pressure increase may be generated be decompression of a spring. Such a spring may exert a pressure on additional liquid, on gas, and/or on a wall of the pressure chamber in order to decrease the volume. As an alternative to using a spring, an electromagnetic actuator may be used. Such an electromagnetic actuator can also be used in order to apply pressure on a liquid substance, on a gas and/or on a wall of the pressure chamber.

According to an implementation, laser-induced bubble generation may be used for increasing the pressure in the pressure chamber. Such bubble generation can evaporate a part of the liquid substance and thus increase the gas volume. Even if the volume of the pressure chamber remains constant, this leads to a pressure increase.

The means described for pressure increase can be used separately or they can be combined.

Especially, the pressure chamber may have a needle portion. The opening may especially be formed at an end of the needle portion. This allows the needle portion to direct the projectile and liquid substance in a specific direction. Especially, the needle portion may have a smaller cross-sectional area and/or a decreasing cross-sectional area towards the opening. Thus, the pressure chamber can have a main part being adapted to store a specific volume, and the cross-sectional area of the needle portion can be adapted to generate the projectile with the intended dimensions.

The invention relates further to an apparatus for injecting a liquid substance into material, especially using a method as described herein. With regard to the method, all embodiments and variations as disclosed herein can be applied. Especially, the method described herein can preferably be applied with the disclosed apparatus.

The apparatus comprises a pressure chamber for providing the liquid substance. The pressure chamber has an opening. The apparatus preferably has a heating device being arranged adjacent to the opening and being configured for at least partially melting a frozen projectile in the pressure chamber adjacent to the opening. The apparatus further preferably comprises a pressurising device for increasing a pressure in the pressure chamber. The apparatus may also have a cooling device. It may have a device that is suitable for both heating and for cooling.

The apparatus can especially be used in order to perform a method as described herein. With regard to the components of the apparatus, reference is made to the description of the method steps, wherein specific components and/or their usage has already been described. The statements given with respect to the method can also be applied for the apparatus.

The pressure chamber may especially be a chamber having only one opening, wherein the opening can be used in order to fill liquid into the pressure chamber, for example, by suction. The opening also serves for forming the projectile and/or releasing the projectile and the liquid substance. Alternatively, the pressure chamber may, for example, have the opening and an additional inlet opening or inlet channel, which may be used separately for filling the pressure chamber with liquid substance, and/or for increasing the pressure. Such an inlet opening or inlet channel may be identical to the pressurising device, or may be comprised by the pressurizing device.

The heating device can especially be used in order to locally heat the frozen projectile, in order to melt a contact portion of the projectile with the opening. This can be used in order to reduce the force holding of the projectile in place.

The pressurising device may be embodied as described above with regard to the method steps. For example, a wall of a pressure chamber may be adapted for varying the volume of the pressure chamber. The pressurising device may exert a force on this part of the wall. For example, the pressurising device may be embodied as a spring or as an electromagnetic actuator. It may also be embodied to put more liquid substance into the pressure chamber. Alternatively, a pressurising device may be embodied as a laser-induced bubble generator, which may be used in order to generate bubbles inside the pressure chamber. Thus, the pressurising device may be embodied as a laser that is able to direct a laser beam into the pressure chamber.

The apparatus may further comprise a cooling device being configured for freezing a part of the liquid substance, or another liquid, in the pressure chamber adjacent to the opening. Thereby, a frozen projectile closing the opening may be formed. A cooling device may especially be separate to the rest of the apparatus, wherein the cooling device may, for example, provide a surface, on which a user of the apparatus may place the opening manually in order to freeze a part of the liquid substance. Despite being separate, it may be regarded as a part of the apparatus. In an alternative implementation, the cooling device may be integrated in a component having the pressure chamber, for example, the cooling device may surround the opening in order to locally cool liquid or liquid substance besides the opening.

It should be noted that the cooling device and the heating device may be embodied in only one component. For example, a thermoelectric element like a Peltier element can be used in order to generate both heat and cold. The same is true for a Carnot cycle device.

Especially, a cooling device may be or may comprise a thermoelectric element, and/or a Carnot cycle device. Such thermoelectric element and/or Carnot cycle devices can especially be used in order to easily generate a specific temperature. As mentioned above, the cooling device and the heating device may be embodied as the same device.

Especially, the heating device may be an electric heater and/or a solenoid. Such electric devices have been proven suitable for generating a local heat.

Especially, the pressurising device may comprise means for introducing more liquid substance, means for introducing compressed gas, means for decreasing a volume of the pressure chamber, a spring configured to increase the pressure in the pressure chamber by decompressing, an electromagnetic actuator, and/or a laser arrangement being configured for laser-induced bubble generation. Such means have been proven suitable in order to increase the pressure in the pressure chamber. Reference is made to the respective descriptions already given above, especially with regard to the apparatus and also with regard to the inventive method.

Especially, it may be regarded as an object to provide a needle-free drug delivery system being capable to deposit a precise amount of liquid at a given depth in biological tissue, avoiding any possibility of cross contamination due to the reuse of the same device in different patients.

Especially, there is proposed here a drug delivery system that uses an ice projectile as a precursor for a liquid jet in a needle-free injection device. Injections are the standard delivery method not only for vaccines, but also for the administration of drugs as part of the treatment of an enormous number of diseases and medical procedures (e.g., 11 billion doses of corona virus vaccines since 2020 up to the date of filing). Each injection produces non-recyclable plastic and metallic waste. The proposal given herein solves the problem of superficial back-splash and also provides a very localized accumulation of liquid at a precise depth into the tissue. The liquid pocket is self-sealed by the tissue. The projectile penetration dynamics in soft tissue are well known and it could be controlled precisely, ranging from epidermal to intramuscular injections. One relevant feature about this invention is the possibility to be used in the whole spectrum of delivery volumes, going from the nanolitres to some millilitres using the same device. For the small volume case the drug can be contained directly in the ice projectile. Also, the implementation disclosed herein does not produce residual waste.

The solution proposed here may be basically regarded as a drug delivery system that uses an ice projectile as a precursor for a liquid jet in a needle-free injection device. In principle, the propulsion of both the projectile and the liquid jet would be produced using one of the variants of the pressurized chamber method. The ice projectile would be produced directly on the outlet of the pressurized reservoir by putting a metallic tip (for instance a microfluidic tube or a flat-end needle) in contact with a "cold piece". The cold piece should have a slot or indent with the same shape as the tip to promote the heat exchange between the tip and the cold piece simply by putting them in direct contact. The cooling system in the cold piece could be given by a "Peltier cell", which produces a temperature difference up to 75° C over its plates when a voltage difference of a few volts is applied to its terminals, for example. Considering projectiles formed from of a nanolitre volume of liquid (that is the volume the needle or microfluidic tip would hold) the formation of the ice projectile would take less than 1 second. As the ice expands inside the tip, it offers a strong liquid seal capable to stand pressures in the liquid reservoir of at least 10 bar (and potentially more). In any case, the release of the ice projectile (and the liquid jet) could be performed by a triggering mechanism that uses a solenoid electrical heater to heat the metallic tip and melt a micrometric boundary layer around the projectile, setting it free. This step would be synchronized to the activation of the pressurizing element. The latter could be given by the opening of a fast electronic valve in a pneumatic system, an electromagnetic actuator, or a compressed spring driver, depending on the specific model of the device. The heater would be also activated simultaneously with the retraction of the cold piece, which is conveniently fixed on a movable arm. All the electronic components are controlled by a programmable chip which also regulates the power supplied to each element based on the data captured by different thermal, pressure, voltage, and position sensors installed on the apparatus.

For example, an overpressure of 9 bar, or between 5 bar and 15 bar, can be used in order to drive the projectile out of the opening. For example, a temperature of -20° C, or between -30° C and -10° C, can be used to freeze liquid substance in order to form the projectile. For example, the opening may have a diameter between 0.2 mm and 0.5 mm. However, it should be noted that these values are only exemplary values that have been proven suitable for typical applications, but that also other values can be used.

The implementation proposed herein can be used in the whole spectrum of delivery volumes, going from nanolitres to some millilitres using the same device, for example. For larger volumes the amount of liquid delivered can be controlled simply by the amount of liquid filling the pressurized reservoir. For the small volume case the drug can be contained directly in the ice projectile, whose volume can be changed by using needles with different internal diameter or by changing the area or the tip in contact with the freezing element (cold piece). The ice can melt inside the body in just a second and then the liquid solution can be absorbed as occurs in a traditional injection. As most of the drugs and vaccines supports freezing without deterioration of the active component, this is a promising technique. Especially, using a frozen projectile ensures that no solid projectile remains in the tissue.

The superior degree of control of the implementation disclosed herein is not only on how much liquid can be delivered, but also on the possibility to regulate the depth of liquid deposition simply by changing the pressure in the reservoir, which in turn would produce a difference in the speed of the projectile.

A regulation of the depth of the injection can be performed by controlling the pressure in the pressurized reservoir, e.g., the pressure chamber. This concept applies to all the possible injection driving system, especially with compressed gas, electromagnetic actuator, loaded spring and/or laser arrangement. The pressure will determine the speed of the ice projectile and then the penetration depth on the soft matter or tissue.

In the following, it is described how pressure and depth of the channel can be controlled.

Compressed gas injection: One can put a pressure regulator connected with a gas electronic valve. The pressure regulator can set the pressure in the pressurized chamber with the liquid to be injected and control the speed of the projectile.

Electromagnetic actuator: In the electromagnetic actuator option a coil can be energized to push the nucleus of the coil. The nucleus of the coil will be attached to a piston which will compress the liquid in the pressurized chamber of the injector. The force exerted by the electromagnet on the piston, and thus the overpressure produced, depends on the current circulating in the coil of the actuator. Controlling the current will result in a control of the pressure in the chamber and then in the speed of the projectile.

Spring: Here the spring can be attached to a piston which will compress the liquid in the pressurized chamber of the injector. Controlling the spring compression will determine the force exerted on the piston, and thus the overpressure produced. Controlling the spring compression will result in a control of the pressure in the chamber and then in the speed of the projectile.

Laser arrangement: In this case, a laser-induced bubble is produced inside the chamber with the liquid. The explosive expansion of the bubble produces a raise in the pressure of the chamber with the liquid that, when synchronized with the release of the ice projectile, will determine its speed. The overpressure produced by the bubble can be controlled directly by setting the laser pulse energy.

A person skilled in the art will derive further features and advantages from the embodiments, which will now be described with reference to the drawings, wherein:
- Fig. 1:: shows an apparatus for injecting a liquid substance according to a first embodiment,
- Fig. 2:: shows the apparatus for injecting a liquid substance in another state,
- Fig. 3:: shows an apparatus according to a second embodiment,
- Fig. 4:: shows an apparatus according to a third embodiment, and
- Fig. 5:: shows an apparatus according to a fourth embodiment.

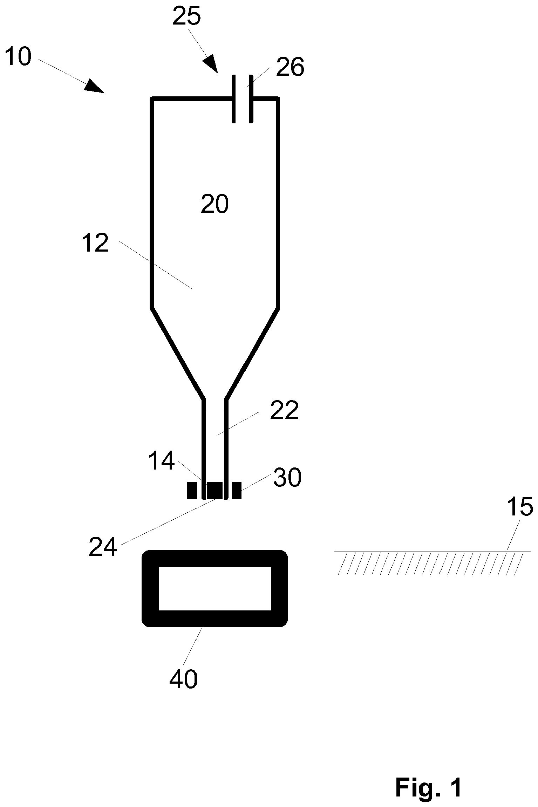

Figure 1 shows an apparatus 10 for injecting a liquid substance into a material 15 according to a first embodiment. As an exemplary material 15, a biological material in the form of a human tissue is shown. The apparatus 10 can especially be used in order to inject a liquid substance 12, for example a liquid drug, into the material 15.

The apparatus 10 comprises a pressure chamber 20, in which the liquid substance 12 to be injected into the material 15 can be provided. The pressure chamber 20 has a needle portion 22, wherein the needle portion 22 is not intended for penetrating the material 15, but is just a portion of the pressure chamber 20, that has a significantly lower cross-sectional area, and it ends with an opening 24.

The pressure chamber 20 further comprises an inlet channel 26, which can be used in order to fill the pressure chamber 20 with liquid substance 12, and in order to increase the pressure in the pressure chamber 20 by filling more liquid substance 12 in the pressure chamber 20.

Besides the opening 24, the apparatus 10 comprises an electric heating device 30, which is embodied as an electric solenoid. The heating device 30 surrounds the needle portion 22 of the pressure chamber 20 immediately adjacent to the opening 24. It can be used in order to locally heat a part of the needle portion 22 adjacent to the opening.

The apparatus 10 further comprises a cooling device 40. The cooling device 40 is separate to the pressure chamber 20. The cooling device 40 is embodied as a thermoelectric element using the Peltier effect, and is configured to present a cold surface towards the pressure chamber 20, especially the needle portion 22. However, it should be noted that other means for cooling, for example, a Carnot cycle, can also be used.

To inject liquid substance 12 in the material 15, the liquid substance 12 is first provided in the pressure chamber 20 by the inlet channel 26. The opening 24 is then manually placed on the cooling device 40, so that a part of the needle portion 22 immediately adjacent to the opening 24 is locally cooled. This leads to freezing of a part of the liquid substance 12, thus forming a frozen projectile 14. The frozen projectile 14 is comprised of the liquid substance 12 and increases in seize, both in axial and radial direction, wherein the increase in seize in radial direction leads to a holding force by friction, which holds the frozen projectile 14 in place.

After generating the projectile by freezing, the pressure chamber 20 is moved to be positioned above the material 15 such that the opening 24 has a short distance to the material 15. For example, 1 mm can be used as a distance. The distance can, for example, be at least 0,5 mm, at least 1 mm, at least 2 mm or at least 3 mm. It can be, for example, be at most 1 mm, at most 2 mm, at most 5 mm, at most 10 or at most 20 mm. These values can be used for all embodiments.

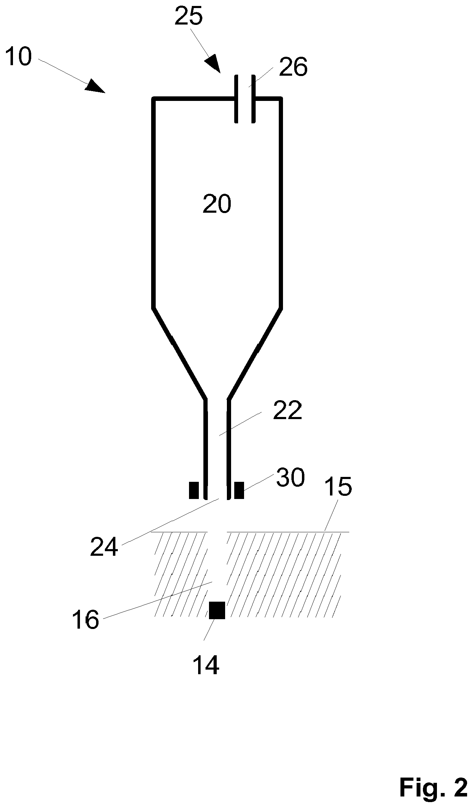

Then, the pressure in the pressure chamber 20 is increased by introducing more liquid substance 12 using the inlet channel 26. Then, the heating device 30 is activated, locally heating the region of the needle portion 22 in which the frozen projectile 14 is located. While doing this, the pressure chamber 20 is located over the material 15, as shown in figure 2.

The heat provided by the heating device 30 locally heats the region around the frozen projectile 14, which leads to a partial melting of contact surfaces of the frozen projectile 14, thus reducing a holding force which holds the frozen projectile 14 in place. When the overpressure in the pressure chamber 20 generates a force in the frozen projectile 14 that is higher than the holding force, the frozen projectile 14 is driven out of the opening 24 by this overpressure. Due to the positioning of the pressure chamber 20 over the material 15, the frozen projectile 14 forms a channel 16 in the material 15, wherein the depth of the channel 16 can be adjusted by suitably setting the overpressure in the pressure chamber 20. The channel 16 provides for the possibility to inject liquid substance 12 into a certain depth of the material 15.

As the inlet channel 26 is used to increase the pressure in the pressure chamber, it can be regarded as a pressurizing device 25.

In an alternative implementation, gas can be introduced using the inlet channel 26. This can also lead to an increase of the pressure inside the pressure chamber 20 and can thus lead to a release of the frozen projectile 14.

After the channel 16 has been formed, the liquid substance 12 present in the pressure chamber 20 is also injected from the opening 24 into the channel 16, so that it penetrates the material 15. The frozen projectile 14 will melt after having formed the channel 16, wherein its melted remaining has the same effect as the injected liquid substance which has not been frozen.

The apparatus 10 thus allows injecting liquid substance like liquid drugs into material like human skin without the need of penetrating the skin with a needle, without any physical contact of the apparatus 10 to the skin, and without any disposable part.

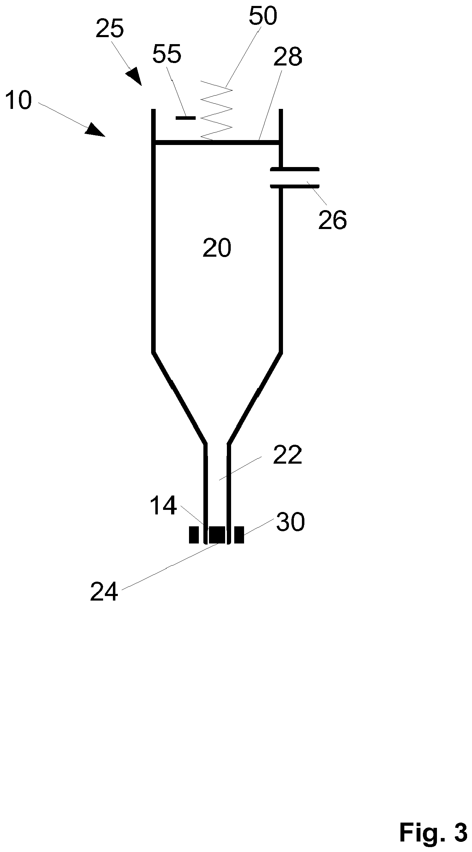

Fig. 3 shows an apparatus 10 according to a second embodiment.

In the second embodiment, the pressurizing device 25 is embodied as a spring 50 acting on a movable wall 28 of the pressure chamber 20. The spring 50 may be secured in a compressed state using a retaining device 55. When the retaining device 55 is activated to release the spring 50, the spring 50 takes a released state, which is shown in Fig. 3. In that state, the movable wall 28 has decreased the volume of the pressure chamber 20, thus increasing the pressure inside the pressure chamber 20. This pressure is an overpressure driving the projectile 14 out of the opening 24.

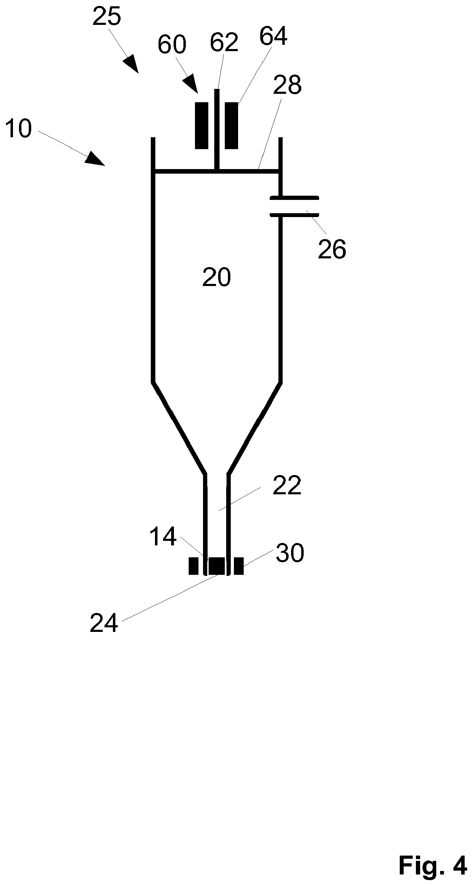

Fig. 4 shows an apparatus 10 according to a third embodiment.

In the third embodiment, the pressurizing device 25 is embodied as an electromagnetic actuator 60 acting on a movable wall 28 of the pressure chamber 20. The electromagnetic actuator 60 comprises an armature 62, that is connected to the movable wall 28 of the pressure chamber 20. It further comprises an electromagnet 64 surrounding the armature 62. If a magnetic field is generated by applying a current through the electromagnet 64, the movable wall 28 is actively moved. Especially, the volume inside the pressure chamber 20 can be increased or decreased. When being decreased, the pressure inside the pressure chamber 20 increases. This pressure is an overpressure driving the projectile 14 out of the opening 24.

It should be noted that the second and third embodiments can be modified such that the pressure chamber 20 does not have a movable wall, but that the spring 50 or the electromagnetic actuator 60 act on a reservoir separate from the pressure chamber 20, thus decreasing the volume of the reservoir. If the reservoir is fluidly connected with the pressure chamber 20, this increases the pressure in the pressure chamber 20.

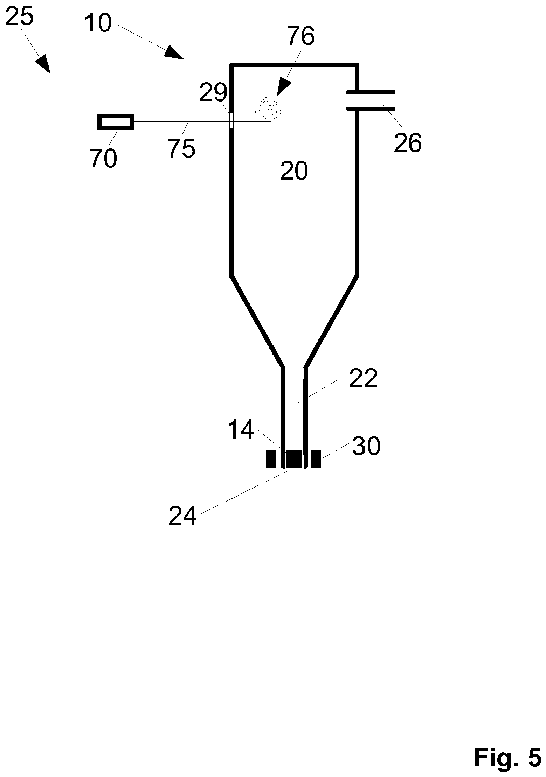

Fig. 5 shows an apparatus 10 according to a fourth embodiment.

In the fourth embodiment, the pressurizing device 25 is embodied as a laser arrangement 70, emitting a laser beam 75 through a window 29 in the pressure chamber 20. When the laser arrangement 70 is activated, the laser beam 75 generates bubbles 76 in the liquid substance 12, i.e., a part of the liquid substance 12 is evaporated. Due to the evaporated liquid, the pressure inside the pressure chamber 20 increases. This pressure is an overpressure driving the projectile 14 out of the opening 24.

Mentioned steps of the inventive method can be performed in the given order. However, they can also be performed in another order, as long as this is technically reasonable. The inventive method can, in an embodiment, for example with a certain combination of steps, be performed in such a way that no further steps are performed. However, also other steps may be performed, including steps that are not mentioned.

It is to be noted that features may be described in combination in the claims and in the description, for example in order to provide for better understandability, despite the fact that these features may be used or implemented independent from each other. The person skilled in the art will note that such features can be combined with other features or feature combinations independent from each other.

References in dependent claims may indicate preferred combinations of the respective features, but do not exclude other feature combinations.

### List of reference signs

- 10: apparatus
- 12: liquid substance
- 14: frozen projectile
- 15: material
- 16: channel
- 20: pressure chamber
- 22: needle portion
- 24: opening
- 25: pressurizing device
- 26: inlet channel
- 28: movable wall
- 29: window
- 30: heating device
- 40: cooling device
- 50: spring
- 55: retaining device
- 60: electromagnetic actuator
- 62: armature
- 64: electromagnet
- 70: laser arrangement
- 75: laser beam
- 76: bubbles

## Claims

1. Method for injecting a liquid substance (12) into a material (15), the method comprising the following steps:
- providing the liquid substance (12) in a pressure chamber (20), the pressure chamber (20) having an opening (24),
- freezing a part of the liquid substance (12), or another liquid, in the pressure chamber (20) adjacent to the opening (24), thereby forming a frozen projectile (14) closing the opening (24),
- applying pressure to the liquid substance (12) in the pressure chamber (20),
- positioning the opening (24) besides the material (15),
- releasing the projectile (14), so that the pressure in the pressure chamber (20) drives the projectile (14) out of the opening (24), the projectile (14) forming a channel (16) in the material (15),
- releasing liquid substance (12) from the pressure chamber (20) out of the opening (24) into the channel (16).

2. Method according to claim 1,
- wherein the freezing of a part of the liquid substance (12) is performed by temporarily placing the opening (24) on a cooling device (40), or by using a cooling device (40) being fixed to the pressure chamber (20).

3. Method according to claim 2,
- wherein the cooling device (40) is or comprises a thermoelectric element or a Carnot cycle apparatus.

4. Method according to one of the preceding claims,
- wherein releasing the projectile (14) comprises at least partially melting the projectile (14) at its contact portion to the pressure chamber (20).
and/or
- wherein releasing the projectile (14) comprises locally heating a region at which the projectile is positioned, so that the projectile (14) is at least partially melted at its contact portion to the pressure chamber (20).

5. Method according to claim 4,
- wherein the partially melting and/or the locally heating is performed by a heating device (30), an electric heater, and/or a solenoid.

6. Method according to one of the preceding claims,
- wherein releasing the projectile (14) comprises pressing the projectile (14) out by overpressure in the pressure chamber (20).

7. Method according to one of the preceding claims,
- wherein the opening (24) is kept out of contact with the material (15) at least during performing the method.

8. Method according to one of the preceding claims,
- wherein the material (15) is biological tissue and the liquid substance (12) is a liquid drug.

9. Method according to one of the preceding claims,
- wherein the pressure is applied to the liquid substance (12) in the pressure chamber (20) by introducing more liquid substance (12), by introducing compressed gas, by decreasing a volume of the pressure chamber (20), by decompression of a spring (50), by an electromagnetic actuator (60), and/or by laser-induced bubble generation.

10. Method according to one of the preceding claims,
- wherein the pressure chamber (20) has a needle portion (22), and
- wherein the opening (24) is formed at an end of the needle portion (22).

11. Apparatus (10) for injecting a liquid substance (12) into material (15) using a method according to one of the preceding claims,
the Apparatus (10) comprising
- a pressure chamber (20) for providing the liquid substance (12), the pressure chamber (20) having an opening (24),
- a heating device (30) being arranged adjacent to the opening (24) and being configured for at least partially melting a frozen projectile (14) in the pressure chamber (20) adjacent to the opening (24), and
- a pressurizing device (25) for increasing a pressure in the pressure chamber (20).

12. Apparatus (10) according to claim 11, further comprising
- a cooling device (40) being configured for freezing a part of the liquid substance (12), or another liquid, in the pressure chamber (20) adjacent to the opening (24), thereby forming a frozen projectile (14) closing the opening (24).

13. Apparatus (10) according to claim 12,
- wherein the cooling device (40) is or comprises a thermoelectric element and/or a Carnot cycle device.

14. Apparatus (10) according to one of claims 11 to 13,
- wherein the heating device (30) is an electric heater and/or a solenoid.

15. Apparatus (10) according to one of claims 11 to 14,
- wherein the pressurizing device (25) comprises means for introducing more liquid substance (12), means for introducing compressed gas, means for decreasing a volume of the pressure chamber (20), a spring (50) configured to increase the pressure in the pressure chamber (20) by decompressing, an electromagnetic actuator (60), and/or a laser arrangement (70) being configured for laser-induced bubble generation.
